# NEW EUROPEAN PATENT SPECIFICATION

(11) **EP 1 066 073 B2**
(45) Date of publication and mention of the opposition decision: **27.02.2008**
(45) Mention of the grant of the patent: 12.06.2002
(21) Application number: 99905106.3
(22) Date of filing: 19.02.1999
(51) Int. Cl.: A61M 15/00, B05D 7/24, B65D 83/14

(54) **Pressurised dispensing containers**
Unter Druck gesetzte Spenderbehälter
Récipients distributeurs pressurisés

(30) Priority: 23.02.1998 GB 9803780; 24.04.1998 GB 9808804; 07.07.1998 GB 9814717
(43) Date of publication of application: 10.01.2001
(60) Divisional application: 01100963.6
(73) Proprietor: GLAXO GROUP LIMITED, Greenford, Middlesex UB6 ONN (GB)
(72) Inventor: WARBY, Richard, John, Emneth, Norfolk PE14 8AF (GB)
(74) Representative: Rice, Jason Neale
(86) International application number: PCT/GB1999/000532
(87) International publication number: WO 1999/042154

(56) References cited:
- EP-A- 0 360 463
- EP-A- 0 469 926
- EP-A- 0 642 992
- WO-A-96/32099
- WO-A-96/32150
- WO-A-96/32151
- WO-A-96/32345
- WO-A-97/32672
- WO-A-97/47347
- US-A- 3 272 442
- US-A- 4 252 848
- US-A- 5 576 068
- "Plasmatechnology", Kirk Othmer Concise Dictionary of Chemical Technology, Vol.19, p.226-258, John Wiley & Sons, 1996
- "Cold Plasma in Materials Fabrication from Fundamentals to Applications", A. Grill, IEEE Press, 1993, Sections 1.6.2, 6.2, 7.1.3, 7.1.4

## Description

This invention relates to improvements in pressurised dispensing containers and particularly those for dispensing a metered dose of medicament.

In metered dose inhalers, an aerosol stream from a pressurised dispensing container is fired towards a patient or user of the inhaler into an air flow. The air flow is created by a user inhaling through a mouthpiece of the inhaler and the medicament is released into this air flow at a point between the air inlet holes and the mouthpiece.

Conventional metering valves for use with pressurised dispensing containers comprise a valve stem co-axially slidable within a valve member defining an annular metering chamber, and outer and inner annular seals operative between the respective outer and inner ends of the valve stem and the valve member to seal the metering chamber therebetween. The valve stem is hollow whereby in a non-dispensing position of the valve stem, the metering chamber is connected to the container and charged with product therefrom. The valve stem is movable against the action of a spring to a dispensing position wherein the metering chamber is isolated from the container and vented to atmosphere for the discharge of product.

Other drug delivery devices include apparatus in which capsules containing a powdered medicament are mechanically opened at a dispensing station where inhaled air subsequently entrains the powder, which is then dispensed through a mouthpiece.

A problem with all such drug delivery devices is that deposition of the medicament, or a solid component from a suspension of a particulate product in a liquid propellant, on the internal surfaces and other components of the devices occurs after a number of operation cycles and/or storage. This can lead to reduced efficiency of operation of the device and of the resulting treatment in that deposition of the product reduces the amount of active drug available to be dispensed.

Some prior art devices rely on the dispenser being shaken in an attempt to dislodge the deposited particles as a result of the movement of a liquid propellant and product mixture. However, whilst this remedy is effective within the body of the container itself, it is not effective for particles deposited on the inner surfaces of the metering chamber. As the size of the chamber is significantly smaller, the restricted flow of fluid in the metering chamber (caused by the tortuosity of the flow path through the chamber) means that the fluid in the metering chamber does not move with enough energy to adequately remove the deposited particles.

One solution is proposed in pending application GB 97211684.0 in which a liner of a material such as fluoropolymer, ceramic or glass is included to line a portion of the wall of a metering chamber in a metering valve. Although this solves the problem of deposition in these types of dispensers, it does require the re-design or modification of moldings and mould tools for producing the valve members to allow for the insertion of the liner.

In each of WO-A-96/32345, WO-A-96/32151, WO-A-96/32150 and WO-A-96/32099 there is disclosed a matered dose inhaler having part or all of its internal surfaces coated with one or more fluorocarbon polymers.

It is an object of the present invention to provide drug delivery devices in general in which the deposition of the product and active drug component is minimised.

According to the invention there is provided a pressurised dispensing container for dispensing a medicament according to claim 1.

The invention also provides a metering valve according to claim 2.

A particular embodiment of the present invention will now be described, by way of example only, with reference to the accompanying drawings, in which:
Figure 1 is a cross-sectional view through an inhaler, with a pressure dispensing container of the present invention; and
Figure 2 is a cross-sectional view of a metering valve for use with a pressure dispensing container.

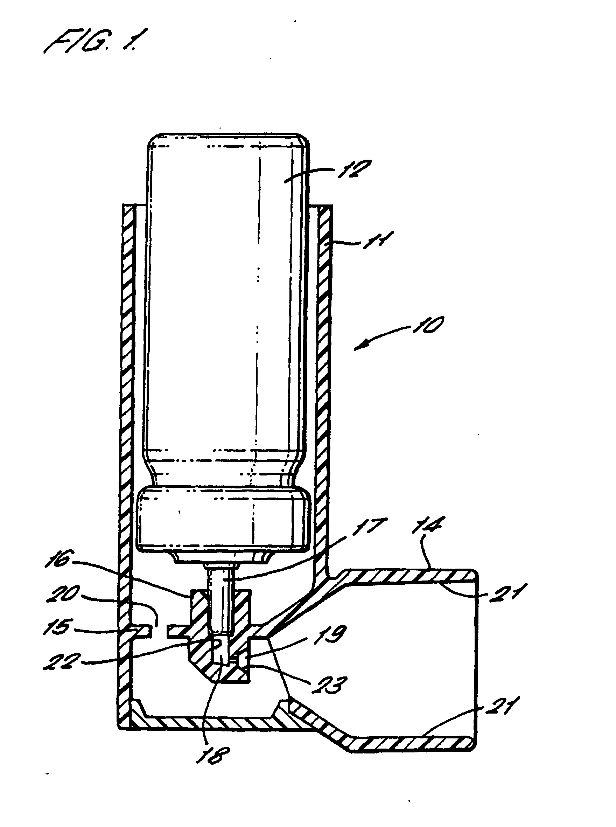

In Figure 1, an inhaler 10 for a product such as a medicament comprises a housing 11 for receiving a pressurised dispensing container 12 of a medicament and a mouthpiece 14 for insertion into the mouth of a user of the inhaler 10.

The container housing 11 is generally cylindrical and open at its upper end. A lower wall 15 of the housing 11 includes an annular socket 16 for receiving the tubular valve stem 17 of the container 12. The socket 16 communicates via a duct 18 ending in an orifice 19 with the mouthpiece 14. The lower wall 15 also has holes 20 for allowing air to flow through the container housing 11 into the mouthpiece 14.

The mouthpiece 14 may be generally circular or shaped to fit the mouth and is connected to or forms a part of the housing 11.

In use, a patient or user holds the inhaler 10, usually in one hand, and applies his mouth to the mouthpiece 14. The user then inhales through the mouthpiece 14 and this creates an airflow through the cylindrical housing 11, from its open end around the dispensing container 12, through the holes 20 and into the mouthpiece 14. After the user has started inhaling through the mouthpiece 14, the container 12 is depressed downwardly onto its stem 17 to release a dose of medicament from the container 12. The dose of medicament is projected by the pressure in the container 12 via the duct 18 and through the orifice 19. It then mixes with the airflow through the mouthpiece 14 and is hence inhaled by the user.

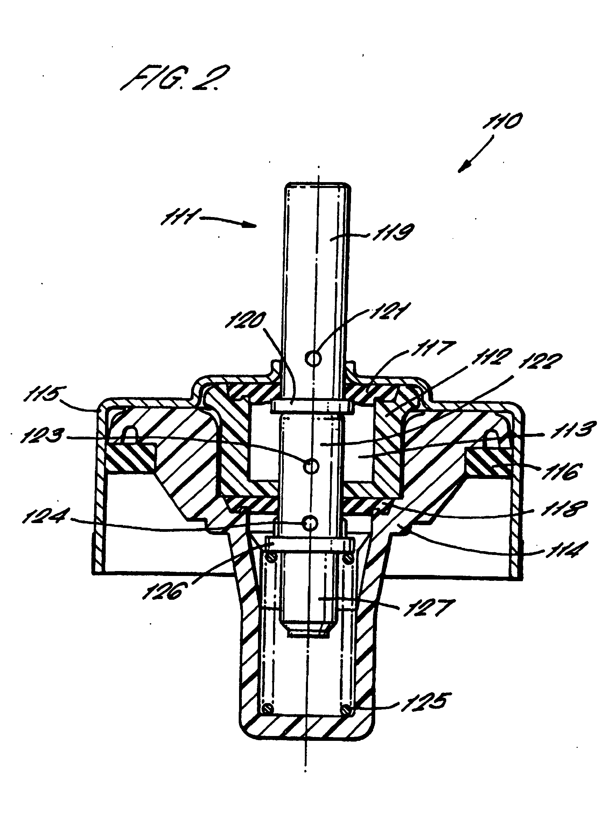

The metering valve 110 illustrated in Figure 2 is another type of drug delivery device or dispenser, and includes a valve stem 111 which protrudes from and is axially slidable within a valve member 112, the valve member 112 and valve stem 111 defining therebetween an annular metering chamber 113. The valve member 112 is located within a valve body 114 which is positioned in a pressurised container (not shown) containing a product to be dispensed. The metering valve 110 is held in position with respect to the container by means of a ferrule 115 crimped to the top of the container and sealing being provided between the valve body 114 and container by an annular gasket 116.

An outer seal 117 and an inner seal 118 of an elastomeric material extend radially between the valve stem 111 and the valve member 112. The outer seal 117 is radially compressed between the valve member 112 and valve stem 111 so as to provide positive sealing contact, the compression being achieved by using a seal which provides an interference fit on the valve stem 111 and/or by the crimping of the ferrule 115 onto the pressurised container during assembly.

The valve stem 111 has an end 119 which protrudes from the valve member 112 and ferrule 115 which is a hollow tube and which is closed off by flange 120 which is located within the metering chamber 113. The hollow end 119 of valve stem 11 includes a discharge port 121 extending radially through the side wall of the valve stem 111. The valve stem 111 further has an intermediate section 122, which is also hollow and defining a central passage and which has a pair of spaced radial ports 123, 124 which are interconnected through a central cavity.

A spring 125 extends between a second flange 126, separating the intermediate section 122 of the valve stem 111 and an inner end 127 of the valve stem 111, and an end of the valve body 114 to bias the valve stem 111 in a non-dispensing position in which the first flange 120 is held in sealing contact with the outer seal 117. The second flange 126 is located outside the valve member 112, but within the valve body 114.

The metering chamber 113 is sealed from the atmosphere by the outer seal 117, and from the pressurised container to which the valve 110 is attached by the inner seal 118. In the illustration of the valve 110 shown in Figure 1 radial ports 123, 124, together with the central cavity in the intermediate section 122 of the valve member 111 connect the metering chamber 113 with the container so that in this non-dispensing condition the metering member 113 will be charged with product to be dispensed.

Upon depression of the valve stem 111 relative to the valve member 112 so that it moves inwardly into the container, the radial port 124 is closed off as it passes through the inner seal 118, thereby isolating the metering chamber 113 from the contents of the pressurised container. Upon further movement of the valve stem 111 in the same direction to a dispensing position the discharge port 121 passes through the outer seal 117 into communication with the metering chamber 113. In this dispensing position the product in the metering chamber 113 is free to be discharge to the atmosphere via the discharge port 121 and the cavity in the hollow end 119 of the valve stem 111.

When the valve stem 111 is released, the biasing of the return spring 125 causes the valve stem 111 to return to its original position. As a result the metering chamber 113 becomes recharged in readiness for further dispensing operations.

The component parts of conventional drug dispensing devices, such as valve members, valve stems, inhaler housings and so on, are generally formed as single mouldings from material such as acetal, polyester or nylon which are prone to the deposition problems described above. Although in some cases it might be possible to include a separate liner of a material such as a fluoropolymer, ceramic or glass to line a portion of the are in which deposition problems occurs, this requires the re-design or modification of mouldings and mould tools so that the components can accommodate such liners.

In the present invention we propose a solution in which the component parts of the pressurised dispensing container are made by conventional tooling and moulds from the traditional materials listed above. They are then subjected to a cold plasma polymerisation treatment with a monomer which is a "hydrophobic" treatment which creates a very thin layer of the plasma polymer on the surface of the component parts which significantly reduces the deposition of active drugs on the relevant surfaces due to factor such as anti-frictional and waterproof characteristics and low surface energy.

The monomer to use in this process is perfluoro-hexane which would creates a thin layer of cold plasma polymerised perfluoro-hexane on the appropriate surface.

The process is known as "cold plasma" treatment as the temperature within the body of the plasma is ambient. Thus thermoplastic materials such as polybutyrene terephthalate (PBT), nylon, acetile and tetrabutyrene terephthalate (TBT) can be treated without fear of thermal damage. The treatment is a vacuum procedure in which the components are placed inside a chamber which is evacuated to less than 0.67 Pa (0.005 Torr). One or more monomers are introduced to the chamber at a controlled rate and a 13.56 MHZ r.f. signal is applied to an external antenna. The plasma is ignited within the chamber and maintained for a given time at the preselected power setting. At the end of the treatment the plasma is extinguished, the chamber flushed and the products retrieved. As a result a thin layer (for example 0.005 to 0.5 microns) of the cold plasma polymerised perfluoro-hexane is intimately bonded to the surface of the component.

In the metering valve of Figure 2, the valve member 112 is treated. In this regard, the valve member 112 alone may be treated. However, additional benefits can be achieved in treating some or all of the other plastic and rubber parts of the valve, including the valve body 114 and the seals 116, 117 and 118. Treatment of the seals 117 and 118 has the additional benefit that friction between the seals 117 and 118 and valve stem 111 is reduced resulting in easier operation of the device. The level of friction between the valve stem 111 and seals 117 and 118 may be further reduced by treatment of the valve stem 111 itself. Such treatment reduces or eliminates the need for silicone emulsions or oils to be applied to the seals 117 and 118 and valve stem 111. Treatment of the seals 116, 117 and 118 also has the benefits of reducing levels of extractibles where the seals are manufactured from elastomeric materials, reducing the permeability of the seals to the propellant in the pressurised dispensing container and reducing the levels of absorption of product onto the surfaces of the seals.

## Claims

1. Pressurised dispensing container for dispensing a medicament, wherein the pressurised dispensing container has:-
a metering valve comprising a valve stem co-axially slidable within a valve member, said valve member and valve stem defining an annular metering chamber, and outer and inner annular seals operative between the respective outer and inner ends of the valve member and the valve stem to seal the annular metering chamber therebetween,
wherein at least a portion of one or more of the interanl surfaces of
components of the metering valve which come into contact with medicament during storage or dispensing has a layer of one or more cold plasma polymerised monomers bonded to at least a portion thereof, wherein said layer is of a cold plasma polymerised fluorinated hydrocarbon, wherein at least a portion of an internal surface of the metering chamber has the layer bonded thereto, wherein the internal surface of the metering chamber is a surface of the valve member and wherein the layer is of cold plasma polymerised perfluoro-hexane.

2. Metering valve for dispensing a medicament for use with a pressurised dispensing container, the valve comprising a valve stem co-axially slidable within a valve member, said valve member and valve stem defining an annular metering chamber, and outer and inner annular seals operative between the respective outer and inner ends of the valve member and the valve stem to seal the annular metering chamber therebetween, **characterized in that** at least a portion of an internal surface of the metering chamber has a layer of one or more cold plasma polymerised monomers bonded thereto, wherein the layer is of a cold plasma polymerised fluorinated hydrocarbon, wherein said internal surface is a surface of the valve member and wherein the layer is of cold plasma polymerised perfluoro-hexane.

## Patentansprüche

1. Unter Druck gesetzter Ausgabebehälter zum Ausgeben eines Medikaments, wobei der unter Druck gesetzte Ausgabebehälter aufweist:
ein Messventil mit einem koaxial innerhalb eines Ventilelements verschiebbaren Ventilschaft, wobei das Ventilelement und der Ventilschaft eine ringförmige Messkammer bestimmen, und äußere und innere ringförmige Dichtungen zwischen den entsprechenden äußeren und inneren Enden des Ventilelements und des Ventilschaftes operativ sind, um die ringförmige Messkammer dazwischen abzudichten,
wobei mindestens ein Bereich einer oder mehrerer der Innenoberflächen von Komponenten des Messventils, die während der Aufbewahrung oder des Ausgebens in Kontakt mit dem Medikament treten, eine Schicht aus einem oder mehreren im kalten Plasma polymerisierten Monomeren aufweist, die mit mindestens einem Bereich derselben verbunden ist, wobei die Schicht ein im kalten Plasma polymerisierter fluorierter Kohlenwasserstoff ist, wobei mindestens ein Bereich einer Innenoberfläche der Messkammer die Schicht mit ihm verbunden aufweist, wobei die Innenoberfläche der Messkammer eine Oberfläche des Ventilelements ist und wobei die Schicht aus im kalten Plasma polymerisiertem Perfluorhexan ist.

2. Messventil zum Ausgeben eines Medikaments, zur Verwendung mit einem unter Druck gesetzten Ausgabebehälter, wobei das Ventil einen koaxial innerhalb eines Ventilelements verschiebbaren Ventilschaft aufweist, das Ventilelement und der Ventilschaft eine ringförmige Messkammer bestimmen, und äußere und innere ringförmige Dichtungen operativ zwischen den entsprechenden äußeren und inneren Enden des Ventilelements und des Ventilschaftes sind, um die ringförmige Messkammer dazwischen abzudichten, **dadurch gekennzeichnet, dass** mindestens ein Bereich einer Innenoberfläche der Messkammer eine damit verbundene Schicht aus einem oder mehreren im kalten Plasma polymerisierten Monomeren aufweist, wobei die Schicht aus im kalten Plasma polymerisierten fluorierten Kohlenwasserstoff ist, wobei die Innenoberfläche eine Oberfläche des Ventilelements ist und wobei die Schicht aus im kalten Plasma polymerisiertem Perfluorhexan ist.

## Revendications

1. Récipient distributeur pressurisé pour distribuer un médicament, où le récipient distributeur pressurisé comprend
un clapet doseur comprenant une tige de clapet coulissable coaxialement à l'intérieure de l'élément de clapet, ledit élément de clapet et ladite tige de clapet définissant une chambre annulaire de dosage et des joints d'étanchéité annulaires externes et internes opérant entre les extrémités externes et internes respectives de l'élément de clapet et de la tige de clapet, afin de sceller hermétiquement la chambre annulaire de dosage entre ceux-ci,
dans lequel au moins une partie d'une ou plusieurs des surfaces internes des composants du clapet de dosage qui viennent en contact avec le médicament pendant l'entreposage ou la distribution, présente une couche d'une ou plusieurs monomères polymérisés à froid à l'aide d'un plasma, liés à au moins une partie de celles-ci, où ladite couche est d'une hydrocarbure fluoré polymérisé à froid à l'aide d'un plasma, où au moins une portion de la surface interne de la chambre de dosage comprend la couche liée à celle-ci, où la surface interne de la chambre de mesure est la surface de l'élément de clapet et où la couche est un prefluorohexane polymérisé à froid à l'aide d'un plasma.

2. Clapet doseur pour distribuer un médicament destiné à une utilisation avec un récipient distributeur pressurisé, le clapet comprenant une tige de clapet coulissable coaxialement à l'intérieure de l'élément de clapet, ledit élément de clapet et ladite tige de clapet définissant une chambre annulaire de dosage et des joints d'étanchéité annulaires externes et internes opérant entre les extrémités externes et internes respectives de l'élément de clapet et de la tige de clapet, afin de sceller hermétiquement la chambre annulaire de dosage entre ceux-ci, **caractérisé en ce que** au moins une portion de la surface interne de la chambre de dosage comprend une couche d'un ou de plusieurs monomères polymérisés à froid à l'aide d'un plasma liés à celle-ci, où la couche est un hydrocarbure fluoré polymérisé à froid à l'aide d'un plasma, où ladite surface interne est la surface de l'élément de clapet et où la couche est un prefluorohexane polymérisé à froid à l'aide d'un plasma.
